(19) Europäisches Patentamt
European Patent Office
Office européen des brevets

(11) EP 4 781 981 A1

(12) **EUROPEAN PATENT APPLICATION**
published in accordance with Art. 153(4) EPC

(43) Date of publication:
**29.07.2026 Bulletin 2026/31**

(21) Application number: **24867453.3**

(22) Date of filing: **18.09.2024**

(51) International Patent Classification (IPC):
***A61K 9/70*** (2006.01) ***A61K 31/496*** (2006.01)
***A61K 47/38*** (2006.01) ***A61K 47/10*** (2017.01)
***A61P 25/24*** (2006.01) ***A61P 25/18*** (2006.01)

(52) Cooperative Patent Classification (CPC):
**A61K 9/70; A61K 31/496; A61K 47/10; A61K 47/32; A61K 47/38; A61P 25/00; A61P 25/18; A61P 25/24**

(86) International application number:
**PCT/CN2024/119447**

(87) International publication number:
**WO 2025/061045 (27.03.2025 Gazette 2025/13)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **22.09.2023 CN 202311227319**

(71) Applicants:
- **Shanghai Aurora Biotechnology Co., Ltd.**
  **Shanghai 201203 (CN)**
- **Shanghai Bocimed Pharmaceutical Research Co., Ltd.**
  **Shanghai 201203 (CN)**
- **Taizhou Bocimed Pharmaceutical Co., Ltd.**
  **Taizhou, Jiangsu 225316 (CN)**

(72) Inventors:
- **YING, Shuhuan**
  **Shanghai 201210 (CN)**
- **FU, Jun**
  **Shanghai 201210 (CN)**
- **LU, Pengcheng**
  **Shanghai 201210 (CN)**
- **LIU, Yuli**
  **Shanghai 201210 (CN)**
- **GUO, Zhen**
  **Shanghai 201210 (CN)**
- **WANG, Tingting**
  **Shanghai 201210 (CN)**

(74) Representative: **Gille Hrabal Partnerschaftsgesellschaft mbB Patentanwälte Brucknerstraße 20 40593 Düsseldorf (DE)**

# (54) BREXPIPRAZOLE ORAL THIN FILM AGENT, PREPARATION METHOD THEREFOR AND USE THEREOF

(57) Provided in the present invention are a brexpiprazole oral thin film agent, a preparation method therefor and the use thereof. The brexpiprazole oral thin film agent comprises the following components: an active drug and a film-forming material, wherein the active drug is 7-[4-(4-benzo[B]thiophene-4-yl-1-piperazine)butoxy]-2(1H)-quinolinone as shown in formula I and/or a pharmaceutically acceptable salt thereof, and the particle size $D_{90}$ of the active drug is ≤70 μm; the film-forming material is one or more of hydroxypropyl methylcellulose, polyvinyl alcohol and hydroxypropyl cellulose; and the brexpiprazole oral thin film agent is free of dimethyl silicone oil. The brexpiprazole oral thin film agent of the present invention has a good dissolution rate, has no gritty feeling after being dissolved in the oral cavity, is uniform in appearance and good in flexibility, does not settle during the formulation of film liquid, and has a content uniformity that meets the requirements.

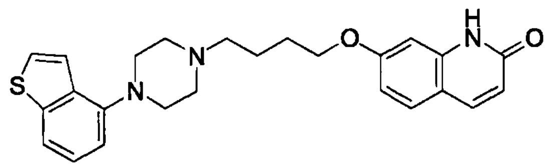

EP 4 781 981 A1

## Description

The present application claims priority to the prior Patent Application No. 202311227319.3 entitled "BREXPIPRAZOLE ORAL THIN FILM AGENT, PREPARATION METHOD THEREFOR AND USE THEREOF" and filed with the China National Intellectual Property Administration on September 22, 2023.

### TECHNICAL FIELD

The present disclosure relates to a brexpiprazole oral thin film formulation, a preparation method therefor, and use thereof.

### BACKGROUND

Brexpiprazole tablets (trade name: REXULTI) were jointly developed by Otsuka Pharmaceutical Co., Ltd. of Japan and Lundbeck A/S of Denmark, and were approved for marketing by the U.S. Food and Drug Administration (FDA) in July 2015. The dosage form is tablets, with strengths of 0.25 mg, 0.5 mg, 1 mg, 2 mg, 3 mg, and 4 mg.

Brexpiprazole is a 5-HT1A receptor and dopamine D2 receptor agonist and a 5-HT2A receptor antagonist, and is clinically used for the treatment of major depressive disorder and schizophrenia. For the treatment of major depressive disorder, the initial dose is 0.5 mg/day or 1 mg/day, which is then increased to a target dose of 2 mg once daily, with a maximum recommended dose of 3 mg/day. For the treatment of schizophrenia, the initial dose is 1 mg/day, the recommended target dose is 2 mg to 4 mg once daily, and the maximum recommended dose is 4 mg/day. Brexpiprazole exhibits broad activity across multiple monoaminergic systems, and shows reduced partial agonist activity at dopamine D2 receptors and increased affinity for specific 5-HT receptors (such as 5-HT1A, 5-HT2A, and 5-HT7), thereby providing improved efficacy and tolerability and reducing adverse reactions such as akathisia, restlessness, or insomnia.

Brexpiprazole (7-[4-(4-benzo[B]thiophen-4-yl-1-piperazine)butoxy]-2(1H)-quinolinone) is a white or off-white crystalline powder, which is practically insoluble in water, has a bitter and numbing irritant taste, and can cause a significant sensation of irritation to the oral mucosa.

Conventional brexpiprazole tablets have to be first disintegrated in the stomach before drug release, resulting in delayed onset of action and thereby limiting bioavailability. Administration is also inconvenient. As a therapeutic drug for psychiatric disorders, the target patient population often shows poor treatment compliance, and behaviors such as treatment refusal, cheeking, and spitting out of the dosage form are likely to occur. Patent document CN105078910A discloses a preparation method for brexpiprazole orally disintegrating tablets, in which brexpiprazole-containing formulations are prepared as freeze-dried orally disintegrating tablets using a lyophilization technique, thereby accelerating disintegration and improving dissolution. However, this technique is relatively complex, requires specialized equipment, results in high production costs, and produces dosage forms that are prone to breakage and unsuitable for transportation, thereby increasing packaging and transportation difficulty. In addition, the dosage form must not come into contact with water during administration, which increases patient requirements and is unfavorable for compliance in patients with schizophrenia.

Patent document CN105395528A discloses a brexpiprazole oral fast-soluble film. However, because brexpiprazole is practically insoluble in water, it is difficult to disperse in a hydrophilic coating solution. During the casting and drying process, the drug is prone to aggregation, thereby affecting the content uniformity of the active ingredient. In addition, patients may experience oral discomfort after administration, which adversely affects compliance.

Therefore, there is an urgent need to develop a brexpiprazole dosage form that is convenient for administration and has stable properties, exhibits good patient compliance, provides high bioavailability, and/or is suitable for industrial-scale production.

### SUMMARY

The present disclosure provides a brexpiprazole oral thin film formulation, comprising the following components: an active pharmaceutical ingredient and a film-forming material, wherein the active pharmaceutical ingredient is 7-[4-(4-benzo[B]thiophen-4-yl-1-piperazine)butoxy]-2(1H)-quinolinone represented by formula I (i.e., brexpiprazole) and/or a pharmaceutically acceptable salt thereof, and the active pharmaceutical ingredient has a particle size $D_{90} \leq 70$ μm; the film-forming material is selected from one or more of hydroxypropyl methylcellulose, polyvinyl alcohol, and hydroxypropylcellulose; the brexpiprazole oral thin film formulation does not comprise dimethyl silicone oil;

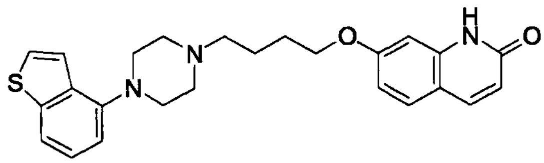

I.

According to an embodiment of the present disclosure, the active pharmaceutical ingredient has a particle size $D_{90} \leq 70.0$ μm, or may have a $D_{90} \leq 65.0$ μm or a $D_{90} \leq 50.0$ μm, e.g., 1.0 μm, 2.0 μm, 3.0 μm, 4.0 μm, 5.0 μm, 6.0 μm, 7.0 μm, 8.0 μm, 9.0 μm, 9.7 μm, 10.0 μm, 11.0 μm, 12.0 μm, 13.0 μm, 14.0 μm, 15.0 μm, 16.0 μm, 17.0 μm, 18.0 μm, 19.0 μm, 20.0 μm, 21.0 μm, 22.0 μm, 23.0 μm, 24.0 μm, 25.0 μm, 26.0 μm, 27.0 μm, 27.8 μm, 29.0 μm, 30.0 μm, 31.0 μm, 32.0 μm, 33.0 μm, 34.0 μm, 35.0 μm, 35.1 μm, 36.0 μm, 37.0 μm, 38.0 μm, 39.0 μm, 40.0 μm, 41.0 μm, 42.0 μm, 43.0 μm, 44.0 μm, 45.0 μm, 45.6 μm, 46.0 μm, 47.0 μm, 48.0 μm, 49.0 μm, 50.0 μm, 55.0 μm, 60.0 μm, or 65.0 μm.

According to an embodiment of the present disclosure, a mass percentage content of the active pharmaceutical ingredient may be 1.0%-50.0% or 4.0%-40.0%, e.g., 40.0%, 35.0%, 30.0%, 25.0%, 23.8%, 20.0%, 16.7%, 15.0%, 13.8%, 10.0%, 8.7%, or 4.3%, wherein the mass percentage content refers to a percentage of the mass of the active pharmaceutical ingredient relative to the total mass of the brexpiprazole oral thin film formulation.

According to an embodiment of the present disclosure, a mass percentage content of the film-forming material may be 30.0%-80.0%, 40.0%-75.0%, or 40.0%-70.0%, e.g., 65.2%, 61.7%, 57.9%, 56.5%, 50.0%, or 47.6%, wherein the mass percentage content refers to a percentage of the mass of the film-forming material relative to the total mass of the brexpiprazole oral thin film formulation.

According to an embodiment of the present disclosure, the brexpiprazole oral thin film formulation may further comprise one or more of a plasticizer, a sweetener, a disintegrant, a filler, and a colorant.

According to an embodiment of the present disclosure, the plasticizer refers to a substance for reducing the glass transition temperature of the film, increasing the plasticity and toughness, and improving the stretching rate, and is selected from one or more of polyethylene glycol, glycerol, propylene glycol, silicone oils other than dimethyl silicone oil, polypropylene glycol, and hexanediol.

According to an embodiment of the present disclosure, a mass percentage content of the plasticizer may be 0-30.0% or 10.0%-30.0%, e.g., 28.0%, 26.0%, 24.1%, 23.8%, 20.8%, 17.4%, 15.0%, 4.0%, or 0, wherein the mass percentage content refers to a percentage of the mass of the plasticizer relative to the total mass of the brexpiprazole oral thin film formulation.

According to an embodiment of the present disclosure, the sweetener refers to a substance for flavoring in the film formulation, and is selected from one or more of aspartame, sucralose, fructose, sucrose, stevioside, glycyrrhizin, an essence, a spice, saccharin, and saccharin sodium.

According to an embodiment of the present disclosure, a mass percentage content of the sweetener may be 0%-10.0% or 5.0%-10.0%, e.g., 8.7%, 8.6%, 4.8%, 0.8%, and 0.7%, wherein the mass percentage content refers to a percentage of the mass of the sweetener relative to the total mass of the brexpiprazole oral thin film formulation.

According to an embodiment of the present disclosure, the disintegrant refers to an auxiliary material for promoting rapid disintegration of a drug into small particles in the gastrointestinal tract, and is selected from one or more of low-substituted hydroxypropylcellulose, crospovidone, croscarmellose sodium, cross-linked sodium carboxymethyl starch, and starch.

According to an embodiment of the present disclosure, a mass percentage content of the disintegrant may be 0-10.0%, e.g., 1.0%, 2.0%, 3.0%, 3.4%, 4.0%, 5.0%, 6.0%, 7.0%, 8.0%, 9.0%, or 0, wherein the mass percentage content refers to a percentage of the mass of the plasticizer relative to the total mass of the brexpiprazole oral thin film formulation.

According to an embodiment of the present disclosure, the filler refers to a solid substance capable of improving the performance of a material, increasing the volume and weight, and reducing the cost of the material when added to the material, and is selected from one or more of microcrystalline cellulose, pregelatinized starch, mannitol, sucrose, glucose, maltose, lactose, sorbitol, xylitol, maltitol, galactitol, erythritol, dextrin, and trehalose.

According to an embodiment of the present disclosure, a mass percentage content of the filler may be 0-60.0%, e.g., 50.0%, 45.0%, 40.0%, 35.0%, 30.4%, 25.0%, 20.0%, 10.0%, or 0, wherein the mass percentage content refers to a percentage of the mass of the filler relative to the total mass of the brexpiprazole oral thin film formulation.

According to an embodiment of the present disclosure, the colorant refers to a substance that is capable of improving the appearance and color of a formulation and can be used for identifying the concentration of the formulation, distinguishing the application method, and reducing the patient's aversion to the administration, and is selected from one or more of titanium dioxide, a pigment, and a lake.

According to an embodiment of the present disclosure, a mass percentage content of the colorant may be 0-5.0%, e.g., 0.1%, 0.5%, 1.0%, 2.0%, or 0, wherein the mass percentage content refers to a percentage of the mass of the

colorant relative to the total mass of the brexpiprazole oral thin film formulation.

According to some embodiments of the present disclosure, the brexpiprazole oral thin film formulation comprises or consists essentially of the following components: brexpiprazole, the film-forming material, a plasticizer, a sweetener, and a filler, wherein the brexpiprazole has a $D_{90} \leq 65.0$ μm.

According to some embodiments of the present disclosure, the brexpiprazole oral thin film formulation comprises or consists essentially of the following components: brexpiprazole, the film-forming material, a plasticizer, a sweetener, and a disintegrant, wherein the brexpiprazole has a $D_{90} \leq 65.0$ μm.

According to some embodiments of the present disclosure, the brexpiprazole oral thin film formulation comprises or consists essentially of the following components: brexpiprazole, the film-forming material, a plasticizer, a colorant, and a disintegrant, wherein the brexpiprazole has a $D_{90} \leq 65.0$ μm.

According to some embodiments of the present disclosure, the brexpiprazole oral thin film formulation comprises or consists essentially of the following components: brexpiprazole, the film-forming material, a plasticizer, a sweetener, and a colorant, wherein the brexpiprazole has a $D_{90} \leq 65.0$ μm.

According to some embodiments of the present disclosure, the brexpiprazole oral thin film formulation comprises or consists essentially of the following components:
4.0%-15.0% brexpiprazole, 50.0%-75.0% hydroxypropyl methylcellulose, 10.0%-30.0% glycerol, 5%-10% sweetener, and 0-0.5% colorant, wherein the brexpiprazole has a $D_{90} \leq 65.0$ μm.

According to an embodiment of the present disclosure, the brexpiprazole oral thin film formulation may be selected from any one of the following formulas:

formula I: 16.7% brexpiprazole, 45.0% hydroxypropyl methylcellulose, 16.7% polyvinyl alcohol, 20.8% glycerol, and 0.8% sucralose, wherein the brexpiprazole has a $D_{90}$ of 27.8 μm;
formula II: 13.8% brexpiprazole, 57.9% hydroxypropyl methylcellulose, 24.1% glycerol, 0.7% stevioside, and 3.5% crospovidone, wherein the brexpiprazole has a $D_{90}$ of 27.8 μm;
formula III: 23.8% brexpiprazole, 47.6% polyvinyl alcohol, 23.8% glycerol, 2.4% stevioside, and 2.4% essence, wherein the brexpiprazole has a $D_{90}$ of 27.8 μm;
formula IV: 4.3% brexpiprazole, 34.8% polyvinyl alcohol, 21.7% hydroxypropylcellulose, 6.5% sucralose, 2.2% essence, and 30.5% microcrystalline cellulose, wherein the brexpiprazole has a $D_{90}$ of 27.8 μm;
formula V: 8.7% brexpiprazole, 65.2% hydroxypropyl methylcellulose, 17.4% glycerol, 4.3% stevioside, 4.3% essence, and 0.1% lake, wherein the brexpiprazole has a $D_{90}$ of 9.7 μm;
formula VI: 8.7% brexpiprazole, 65.2% hydroxypropyl methylcellulose, 17.4% glycerol, 4.3% stevioside, 4.3% essence, and 0.1% lake, wherein the brexpiprazole has a $D_{90}$ of 16.5 μm;
formula VII: 8.7% brexpiprazole, 65.2% hydroxypropyl methylcellulose, 17.4% glycerol, 4.3% stevioside, 4.3% essence, and 0.1% lake, wherein the brexpiprazole has a $D_{90}$ of 27.8 μm;
formula VIII: 8.7% brexpiprazole, 65.2% hydroxypropyl methylcellulose, 17.4% glycerol, 4.3% stevioside, 4.3% essence, and 0.1% lake, wherein the brexpiprazole has a $D_{90}$ of 35.1 μm;
formula IX: 8.7% brexpiprazole, 65.2% hydroxypropyl methylcellulose, 17.4% glycerol, 4.3% stevioside, 4.3% essence, and 0.1% lake, wherein the brexpiprazole has a $D_{90}$ of 45.6 μm; and
formula X: 40.0% brexpiprazole, 50.0% polyvinyl alcohol, 4.0% glycerol, 2.0% titanium dioxide, and 4.0% crospovidone, wherein the brexpiprazole has a $D_{90}$ of 45.6 μm.

According to an embodiment of the present disclosure, the brexpiprazole oral thin film formulation has a thickness of 10 μm-100 μm, e.g., 20 μm, 30 μm, 40 μm, 50 μm, 60 μm, 70 μm, 80 μm, or 90 μm.

The present disclosure further provides a preparation method for the brexpiprazole oral thin film formulation, and the preparation method comprises the following steps:

1) dissolving one or more of water-soluble excipients such as the plasticizer, the sweetener, the disintegrant, the filler, and the colorant in purified water, and adding the film-forming material and dissolving the film-forming material;
2) mixing insoluble excipients in a formulation with the solution obtained in step 1) to obtain a suspension;
3) adding the active pharmaceutical ingredient into the solution obtained in step 2), and mixing uniformly;
4) defoaming the suspension obtained in step 3) to obtain a defoamed suspension; and
5) coating a substrate with the defoamed suspension obtained in step 4), drying, and forming a film to obtain the brexpiprazole oral thin film formulation.

According to an embodiment of the present disclosure, in step 2, the mixing is preferably a homogeneous mixing.

The present disclosure further provides use of the brexpiprazole oral thin film formulation for manufacturing a medicament for the treatment of a central nervous system disease.

According to an embodiment of the present disclosure, the central nervous system disease may be major

depressive disorder or schizophrenia.

The present disclosure further provides a method for the treatment of a central nervous system disease, and the method comprises administering to a patient in need thereof a therapeutically effective amount of the brexpiprazole oral thin film formulation.

The preferred conditions described above may be combined arbitrarily to obtain preferred embodiments of the present disclosure without departing from the general knowledge in the art.

The reagents and starting materials used in the present disclosure are commercially available.

Beneficial Effects of Present Disclosure:

The brexpiprazole oral thin film formulation of the present disclosure at least has one of the following advantages: The brexpiprazole oral thin film formulation exhibits good dissolution performance and rapid disintegration, does not produce a gritty sensation after dissolving in the oral cavity, has a uniform appearance and good flexibility, and shows stable properties (such as stable chemical properties, stable dissolution performance, stable mechanical strength, and/or stable folding endurance). In addition, no sedimentation occurs during preparation of the film-forming solution, and the content uniformity meets the requirements.

Definitions and Description

The term "more" refers to two or more, e.g., two, three or more.

The term "consisting essentially of" means that the sum of the mass of these components is not less than 85%, e.g., not less than 90%, illustratively 90%, 91%, 92%, 93%, 94%, 95%, 96%, 97%, 98%, 99%, or 100% of the total mass of the brexpiprazole oral thin film formulation.

The term "patient" refers to any animal including mammals, preferably mice, rats, other rodents, rabbits, dogs, cats, pigs, cattle, sheep, horses, or primates, and most preferably humans.

The term "therapeutically effective amount" refers to the amount of the active compound or drug that causes a biological or medical response that researchers, veterinarians, physicians, or other clinicians are looking for in tissues, systems, animals, individuals, or humans.

## DETAILED DESCRIPTION

The technical solutions of the present disclosure are further described in detail below with reference to specific examples. It will be appreciated that the following examples are merely exemplary illustrations and explanations of the present disclosure and should not be construed as limiting the claimed scope of the present disclosure. All techniques implemented on the basis of the content described above of the present disclosure fall within the claimed scope of the present disclosure.

Unless otherwise stated, the starting materials and reagents used in the following examples are all commercially available products or can be prepared by using known methods or can be selected according to the commodity instructions.

Examples 1-10: Formulations are shown below (% in the table denotes weight percentages)

| Example | Example 1 | Example 2 | Example 3 | Example 4 | Example 5 |
|---|---|---|---|---|---|
| Brexpiprazole ($D_{90}$ = 9.7 μm) | / | / | / | / | 1.00 g (8.7%) |
| Brexpiprazole ($D_{90}$ = 27.8 μm) | 2.00 g (16.7%) | 2.00 g (13.8%) | 2.00 g (23.8%) | 1.00 g (4.3%) | / |
| Hydroxypropyl methylcellulose | 5.40 g (45.0%) | 8.40 g (57.9%) | / | / | 7.50 g (65.2%) |
| Polyvinyl alcohol | 2.00 g (16.7%) | / | 4.00 g (47.6%) | 8.00 g (34.8%) | / |
| Hydroxypropylcellulose | / | / | / | 5.00 g (21.7%) | / |

(continued)

| Example | Example 1 | Example 2 | Example 3 | Example 4 | | Example 5 |
|---|---|---|---|---|---|---|
| Glycerol | 2.50 g (20.8%) | 3.50 g (24.1%) | 2.00 g (23.8%) | / | | 2.00 g (17.4%) |
| Stevioside | / | 0.10 g (0.7%) | 0.20 g (2.4%) | / | | 0.50 g (4.3%) |
| Sucralose | 0.10 g (0.8%) | / | / | 1.50 g (6.5%) | | / |
| Essence | / | / | 0.20 g (2.4%) | 0.50 g (2.2%) | | 0.50 g (4.3%) |
| Lake | / | / | / | / | | 0.01g (0.1%) |
| Crospovidone | / | 0.50 g (3.5%) | / | / | | / |
| Microcrystalline cellulose | / | / | / | 7.00 g (30.5%) | | / |
| Purified water* | 50.00 g | 70.00 g | 30.00 g | 65.00 g | | / |
| **Example** | **Example 6** | **Example 7** | **Example 8** | **Example 9** | **Example 9'** | **Example 10** |
| Brexpiprazole ($D_{90}$ = 9.7 μm) | / | / | / | / | / | / |
| Brexpiprazole ($D_{90}$ = 16.5 μm) | 1.00 g (8.7%) | / | / | / | / | / |
| Brexpiprazole ($D_{90}$ = 27.8 μm) | / | 1.00 g (8.7%) | / | / | / | / |
| Brexpiprazole ($D_{90}$ = 35.1 μm) | / | / | 1.00 g (8.7%) | / | / | / |
| Brexpiprazole ($D_{90}$ = 45.6 μm) | / | / | / | 1.00 g (8.7%) | 1.00 g (7.7%) | 2.00 g (40.0%) |
| Hydroxypropyl methylcellulose | 7.50 g (65.2%) | 7.50 g (65.2%) | 7.50 g (65.2%) | 7.50 g (65.2%) | 7.50 g (57.6%) | |
| Polyvinyl alcohol | / | / | / | / | / | 2.50 g (50.0%) |
| Glycerol | 2.00 g (17.4%) | 2.00 g (17.4%) | 2.00 g (17.4%) | 2.00 g (17.4%) | 2.00 g (15.4%) | 0.20 g (4.0%) |
| Stevioside | 0.50 g (4.3%) | 0.50 g (4.3%) | 0.50 g (4.3%) | 0.50 g (4.3%) | 2.00 g (15.4%) | / |
| Essence | 0.50 g (4.3%) | 0.50 g (4.3%) | 0.50 g (4.3%) | 0.50 g (4.3%) | 0.50 g (3.8%) | / |
| Lake | 0.01g (0.1%) | 0.01g (0.1%) | 0.01g (0.1%) | 0.01g (0.1%) | 0.01g (0.1%) | / |
| Titanium dioxide | / | / | / | / | / | 0.10 g (2.0%) |
| Crospovidone | / | / | / | / | / | 0.20 g (4.0%) |
| Purified water* | 60.00 g | 60.00 g | 60.00 g | 50.00 g | 60.00 g | 10.0 g |
| Purified water* is a solvent and a wetting agent that are removed during the process. | | | | | | |

Preparation method:

1) Water-soluble excipients such as a plasticizer, a sweetener, and a disintegrant were dissolved in purified water, and the film-forming material was added and then dissolved;
2) insoluble excipients in a formulation were mixed with the solution obtained in step 1) to obtain a suspension;
3) the active ingredient was added into the solution obtained in step 2), and the mixture was mixed uniformly;
4) the suspension obtained in step 3) was defoamed to obtain a defoamed suspension; and
5) a substrate was coated with the defoamed suspension obtained in step 4), followed by drying and forming a film, to obtain the brexpiprazole oral thin film formulation.

Comparative Examples 1-3

The oral thin film formulation of Comparative Example 1 was prepared by repeating procedures of Example 4 of the Invention Patent Application No. CN115192549A, and the oral thin film formulation of Comparative Example 2 was prepared by replacing the dimethyl silicone oil in Comparative Example 1 with glycerol. The formulations are shown below (% in the table denotes weight percentages)

| Comparative Example | Comparative Example 1 | Comparative Example 2 | Comparative Example 3 |
|---|---|---|---|
| Brexpiprazole ($D_{90}$ = 27.8 μm) | 5.00 g (25.0%) | 5.00 g (25.0%) | / |
| Brexpiprazole ($D_{90}$ = 35.1 μm) | / | / | 5.00 g (25.0%) |
| Hydroxypropyl methylcellulose | 2.88 g (14.4%) | 2.88 g (14.4%) | 2.88 g (14.4%) |
| Polyvinyl alcohol | 8.00 g (40.0%) | 8.00 g (40.0%) | 8.00 g (40.0%) |
| Glycerol | 4.00 g (20.0%) | 4.10 g (20.5%) | 4.00 g (20.0%) |
| Dimethyl silicone oil | 0.10 g (0.5%) | / | 0.1 g (0.5%) |
| Sucralose | 0.02 g (0.1%) | 0.02 g (0.1%) | 0.02 mg (0.1%)) |
| Purified water* | 160.00 g | 160.00 g | 160.00 g |
| Purified water* is a solvent and a wetting agent that are removed during the process. | | | |

During the preparation of the oral thin film formulation of Comparative Example 1, it was found that after dimethyl silicone oil was added to the formulation (Comparative Example 1), the drug film spontaneously peeled off from the substrate during the drying process. Since manual cutting was adopted at the small-scale experimental stage, this phenomenon was not observed. However, after the formulation was processed in large-scale equipment, it was found that subsequent cutting and packaging processes for the oral thin film formulation of Comparative Example 1 could not be accurately completed due to the peeling issue, which was unfavorable for large-scale production.

However, in Comparative Example 2, after the dimethyl silicone oil was replaced with glycerol, the problem described above could be effectively avoided.

The applicants prepared the oral thin film formulation of Comparative Example 3 by repeating the procedures of Example 5 of the Invention Patent No. CN115192549A. The formulation is as shown above (% in the table denotes weight percentages), and the formulation was prepared using the process disclosed in CN115192549A and the process of the examples of the present disclosure.

The results showed that, in CN115192549A, brexpiprazole and the film-forming material were dissolved simultaneously. When the particle size of brexpiprazole was excessively large, film formation occurred during dissolution of the film-forming material, causing brexpiprazole to be entrapped within the film-forming matrix, which was unfavorable for dispersion. When the $D_{90}$ of brexpiprazole was greater than 30 μm, the resulting solution exhibited relatively poor homogeneity. However, in the present disclosure, by optimizing the process, the film-forming material is dissolved first and brexpiprazole is subsequently added, such that brexpiprazole can be well dispersed under the action of a high-speed shear mixer. Therefore, even when the $D_{90}$ of brexpiprazole is greater than 30 μm, qualified finished products can still be prepared.

Test Example

Content detection method: Octadecylsilane-bonded silica gel was used as the filler; phosphate buffer-acetonitrile (70:30) was used as the mobile phase; the flow rate was 1.0 mL/min; the column temperature was 40 °C; the detection wavelength was 275 nm; the injection volume was 10 μL; and the run time was 10 min.

Test method for related substances: Octadecylsilane-bonded silica gel was used as the filler; phosphate buffer was used as mobile phase A; phosphate buffer-acetonitrile (35:65) was used as mobile phase B; gradient elution was performed, and the detection wavelength was 275 nm; the flow rate was 1.0 mL/min; the column temperature was 40 °C; and the injection volume was 50 μL.

Dissolution test method other than those in Table 3: 900 mL of a pH 4.3 acetate buffer containing 0.1% polysorbate 20 was used as the dissolution medium; the rotation speed was 50 rpm; and samples were collected after 30 min.

Mechanical strength test: A texture analyzer (model: R brexpiprazole d $TA^+$; manufacturer: Shanghai Tengba Instrument Technology Co., Ltd.) was used to test the mechanical strength of the brexpiprazole oral soluble film formulation.

A tensile test module was installed after startup, force calibration and height calibration were performed, and the height was set to 2 mm.

The thickness and width of the oral soluble films were measured using a thickness gauge and a ruler, and the cross-sectional areas ($mm^2$) of the oral soluble films were calculated.

The oral soluble films after the thickness and width were measured were fixed on a texture analyzer such that the measured cross section was perpendicular to the stretching direction of the instrument, and the maximum force and the height of the sample were recorded.

$$\text{Tensile strength (MPa)} = \text{maximum force (gf)} \div 102 \div \text{cross-sectional area (mm}^2\text{)}.$$

$$\text{Percentage elongation} = \text{maximum force distance (mm)} \div \text{sample height (mm)} \times 100\%.$$

Folding endurance: Folding endurance refers to the number of folds at the same position until breakage or the appearance of obvious creases. Specifically, the oral soluble films were folded 180° along the center and then unfolded, and the folding operation was repeated to observe whether the oral soluble films broke. The brexpiprazole oral thin film formulations prepared according to Examples 1-10 and Comparative Examples 2 and 3 were tested for appearance, content, content uniformity, related substances, dissolution, dissolution profiles, mechanical properties, and disintegration time.

Table 1. Detection results of appearance, content, content uniformity, and dissolution of brexpiprazole oral thin film formulation

| Example | Example 1 | Example 2 | Example 3 | | Example 4 | Example 5 | Example 6 |
|---|---|---|---|---|---|---|---|
| Brexpiprazole $D_{90}$ | 27.8 μm | 27.8 μm | 27.8 μm | | 27.8 μm | 9.7 μm | 16.5 μm |
| Appearance | White uniform sheet-like thin film | White uniform sheet-like thin film | White uniform sheet-like thin film | | White uniform sheet-like thin film | Red uniform sheet-like thin film | Red uniform sheet-like thin film |
| Content | 99.8% | 98.7% | 100.2% | | 100.3% | 99.9% | 98.7% |
| Content uniformity | Meet the requirements | Meet the requirements | Meet the requirements | | Meet the requirements | Meet the requirements | Meet the requirements |
| Dissolution | 95% | 92% | 90% | | 90% | 95% | 89% |
| Example | Example 7 | Example 8 | Example 9 | Example 9' | Example 10 | Comparative Example 2 | Comparative Example 3 |
| Brexpiprazole $D_{90}$ | 27.8 μm | 35.1 μm | 45.6 μm | 45.6 μm | 45.6 μm | 27.8 μm | 35.1 μm |
| Appearance | Red uniform sheet-like thin film | Red uniform sheet-like thin film | Red sheet-like thin film | Red sheet-like thin film with solid particles on the surface | White uniform sheet-like thin film | White uniform sheet-like thin film | White sheet-like thin film with solid particles on the surface |
| Content | 101.3% | 101.0% | 100.3% | N/A | 98.2% | 99.3% | N/A |
| Content uniformity | Meet the requirements | Meet the requirements | Meet the requirements | Do not meet the requirements | Meet the requirements | Meet the requirements | Do not meet the requirements |
| Dissolution | 90% | 85% | 84% | N/A | 88% | 91% | N/A |

Based on the results described above, when the $D_{90}$ of the brexpiprazole oral thin film formulations was ≤ 70.0 μm, oral thin film formulations with relatively good appearance and content uniformity could be prepared, and the content and dissolution both met the requirements.

1. Test for related substances in brexpiprazole oral thin film formulation

Table 2. Detection of related substances in brexpiprazole oral thin film formulation

| Example | Example 1 | Example 2 | Example 3 | Example 4 | Example 5 | Example 6 | Example 7 | Example 8 | Example 9 | Example 10 | Comparative Example 2 |
|---|---|---|---|---|---|---|---|---|---|---|---|
| Related substance | IM-E: undetectable<br>IM-30: undetectable<br>IM-56: undetectable<br>Maximum unknown individual impurity: 0.08%<br>Total impurity: 0.10% | IM-E: 0.05%<br>IM-30: undetectable<br>IM-56: undetectable<br>Maximum unknown individual impurity: 0.08%<br>Total impurity: 0.20% | IM-E: 0.10%<br>IM-30: undetectable<br>IM-56: undetectable<br>Maximum unknown individual impurity: 0.05%<br>Total impurity: 0.24% | IM-E: undetectable<br>IM-30: undetectable<br>IM-56: undetectable<br>Maximum unknown individual impurity: 0.05%<br>Total impurity: 0.12% | IM-E: undetectable<br>IM-30: undetectable<br>IM-56: undetectable<br>Maximum unknown individual impurity: 0.05%<br>Total impurity: 0.12% | IM-E: undetectable<br>IM-30: undetectable<br>IM-56: undetectable<br>Maximum unknown individual impurity: 0.05%<br>Total impurity: 0.05% | IM-E: undetectable<br>IM-30: undetectable<br>IM-56: undetectable<br>Maximum unknown individual impurity: 0.05%<br>Total impurity: 0.08% | IM-E: undetectable<br>IM-30: undetectable<br>IM-56: undetectable<br>Maximum unknown individual impurity: 0.05%<br>Total impurity: 0.10% | IM-E: undetectable<br>IM-30: undetectable<br>IM-56: undetectable<br>Maximum unknown individual impurity: 0.07%<br>Total impurity: 0.12% | IM-E: undetectable<br>IM-30: undetectable<br>IM-56: undetectable<br>Maximum unknown individual impurity: 0.08%<br>Total impurity: 0.15% | IM-E:undetectable<br>IM-30:undetectable<br>IM-56:undetectable<br>Maximum unknown individual impurity: 0.06%<br>Total impurity: 0.18% |

Based on the results described above, the related substances in the brexpiprazole oral thin film formulations prepared in Examples 1-10 met the requirements.

2. Dissolution profile test for brexpiprazole oral thin film formulation

Dissolution method: According to General Chapter 0931, Chinese Pharmacopoeia, Volume IV, 2020 Edition, Method II: Paddle Method, using a sinker: the rotation speed was 50 rpm, the volume of the dissolution medium was 900 mL, and the temperature was 37 °C.

Table 3. Detection of dissolution profile of brexpiprazole oral thin film formulation

| **Hydrochloric acid solution at pH 1.2** | **5 min** | **10 min** | **15 min** | **20 min** | **30 min** | **45 min** | **60 min** | **90 min** | **120 min** | **Limit** |
|---|---|---|---|---|---|---|---|---|---|---|
| Control drug (trade name: REXULTI) | 33% | 40% | 46% | 48% | 55% | 60% | 67% | 72% | 74% | 83% |
| Example 1 | 41% | 45% | 49% | 52% | 63% | 68% | 70% | 76% | 80% | 85% |
| Example 2 | 43% | 46% | 50% | 55% | 60% | 65% | 71% | 77% | 79% | 83% |
| Example 3 | 42% | 47% | 47% | 52% | 59% | 64% | 70% | 78% | 82% | 88% |
| Example 4 | 44% | 46% | 47% | 51% | 60% | 65% | 68% | 75% | 78% | 85% |
| Example 5 | 55% | 62% | 69% | 75% | 80% | 85% | 86% | 88% | 88% | 89% |
| Example 6 | 50% | 57% | 65% | 68% | 70% | 74% | 80% | 84% | 86% | 88% |
| Example 7 | 29% | 38% | 47% | 51% | 57% | 63% | 69% | 76% | 79% | 84% |
| Example 8 | 26% | 32% | 35% | 40% | 45% | 57% | 64% | 72% | 75% | 84% |
| Example 9 | 24% | 19% | 31% | 38% | 42% | 51% | 60% | 68% | 72% | 82% |
| Example 10 | 19% | 24% | 29% | 36% | 42% | 48% | 55% | 68% | 71% | 83% |
| Comparative Example 2 | 34% | 45% | 48% | 51% | 57% | 60% | 64% | 68% | 70% | 75% |
| Acetate buffer at pH 4.3 + 0.1% Tween 20 | **5 min** | **10 min** | **15 min** | **20 min** | **30 min** | **45 min** | **60 min** | **Limit** | | |
| Control drug (trade name: REXULTI) | 60% | 74% | 80% | 83% | 88% | 89% | 91% | 94% | | |
| Example 1 | 71% | 82% | 89% | 91% | 94% | 100% | 99% | 100% | | |
| Example 2 | 74% | 85% | 94% | 95% | 98% | 98% | 100% | 101% | | |
| Example 3 | 72% | 83% | 90% | 94% | 97% | 100% | 99% | 101% | | |
| Example 4 | 75% | 85% | 90% | 96% | 99% | 99% | 101% | 101% | | |
| Example 5 | 77% | 89% | 94% | 97% | 100% | 100% | 100% | 101% | | |
| Example 6 | 66% | 80% | 83% | 85% | 88% | 88% | 91% | 93% | | |
| Example 7 | 56% | 70% | 78% | 80% | 86% | 89% | 90% | 92% | | |
| Example 8 | 53% | 67% | 75% | 79% | 85% | 87% | 89% | 94% | | |
| Example 9 | 50% | 63% | 71% | 76% | 81% | 85% | 87% | 96% | | |
| Example 10 | 48% | 55% | 67% | 75% | 80% | 83% | 88% | 95% | | |
| Comparative Example 2 | 64% | 76% | 82% | 88% | 98% | 99% | 100% | 100% | | |

The data showed that the dissolution profiles of the brexpiprazole oral thin film formulations of Examples 1-10 were slightly different from that of the control drug (trade name: REXULTI).

3. Mechanical strength test for brexpiprazole oral thin film formulation

The test results of the mechanical strength of the brexpiprazole oral soluble film formulations are shown in Table 4.

Table 4. Mechanical strength test for brexpiprazole oral soluble films prepared in Examples 1-10 and Comparative Example 2

| | Thickness/mm | Width/mm | Cross-sectional area/mm$^2$ | Maximum force/gf | Tensile strength/MPa | Average tensile strength/MPa | Maximum force distance/mm | Sample height/mm | Percentage elongation |
|---|---|---|---|---|---|---|---|---|---|
| Example 1 | 0.05 | 20 | 1.0 | 2854.6 | 28.0 | 26.7 | 0.4 | 2.1 | 19% |
| | 0.05 | 20 | 1.0 | 2931.6 | 28.7 | | 0.4 | 2.1 | 20% |
| | 0.05 | 20 | 1.0 | 2920.4 | 28.6 | | 0.4 | 2.1 | 19% |
| | 0.05 | 20 | 1.0 | 2025.0 | 19.9 | | 0.5 | 2.1 | 23% |
| | 0.05 | 20 | 1.0 | 2759.4 | 27.1 | | 0.4 | 2.1 | 18% |
| | 0.05 | 20 | 1.0 | 2828.8 | 27.7 | | 0.4 | 2.1 | 19% |
| Example 2 | 0.05 | 20 | 1.0 | 2003.3 | 19.6 | 23.2 | 0.5 | 2.1 | 21% |
| | 0.05 | 20 | 1.0 | 2702.4 | 26.5 | | 0.4 | 2.1 | 18% |
| | 0.05 | 20 | 1.0 | 2279.0 | 22.3 | | 0.3 | 2.1 | 14% |
| | 0.06 | 20 | 1.2 | 1827.3 | 14.9 | | 0.5 | 2.1 | 25% |
| | 0.05 | 20 | 1.0 | 2820.3 | 27.7 | | 0.4 | 2.1 | 19% |
| | 0.05 | 20 | 1.0 | 2877.9 | 28.2 | | 0.4 | 2.1 | 19% |
| Example 3 | 0.05 | 20 | 1.0 | 1997.9 | 19.6 | 18.6 | 0.3 | 2.1 | 15% |
| | 0.05 | 20 | 1.0 | 1965.4 | 19.3 | | 0.4 | 2.1 | 20% |
| | 0.05 | 20 | 1.0 | 1819.8 | 17.8 | | 0.4 | 2.1 | 18% |
| | 0.05 | 20 | 1.0 | 1999.6 | 19.6 | | 0.4 | 2.1 | 18% |
| | 0.05 | 20 | 1.0 | 1897.6 | 18.6 | | 0.4 | 2.1 | 19% |
| | 0.06 | 20 | 1.2 | 2035.8 | 16.6 | | 0.5 | 2.1 | 23% |
| Example 4 | 0.05 | 20 | 1.0 | 1805.0 | 17.7 | 17.2 | 0.5 | 2.1 | 24% |
| | 0.05 | 20 | 1.0 | 2009.3 | 19.7 | | 0.5 | 2.1 | 23% |
| | 0.05 | 20 | 1.0 | 1658.4 | 16.3 | | 0.4 | 2.1 | 17% |
| | 0.06 | 20 | 1.2 | 1815.1 | 14.8 | | 0.4 | 2.1 | 20% |
| | 0.05 | 20 | 1.0 | 2029.2 | 19.9 | | 0.5 | 2.1 | 23% |
| | 0.05 | 20 | 1.0 | 1541.8 | 15.1 | | 0.4 | 2.1 | 17% |

(continued)

| | Thickness/mm | Width/mm | Cross-sectional area/mm$^2$ | Maximum force/gf | Tensile strength/MPa | Average tensile strength/MPa | Maximum force distance/mm | Sample height/mm | Percentage elongation |
|---|---|---|---|---|---|---|---|---|---|
| Example 5 | 0.05 | 20 | 1.0 | 1911.8 | 18.7 | 18.1 | 0.4 | 2.1 | 18% |
| | 0.05 | 20 | 1.0 | 1762.6 | 17.3 | | 0.3 | 2.1 | 15% |
| | 0.05 | 20 | 1.0 | 1839.0 | 18.0 | | 0.4 | 2.1 | 17% |
| | 0.05 | 20 | 1.0 | 1774.2 | 17.4 | | 0.3 | 2.1 | 15% |
| | 0.05 | 20 | 1.0 | 1849.5 | 18.1 | | 0.4 | 2.1 | 18% |
| | 0.05 | 20 | 1.0 | 1932.5 | 18.9 | | 0.4 | 2.1 | 19% |
| Example 6 | 0.05 | 20 | 1.0 | 1937.2 | 19.0 | 18.1 | 0.3 | 2.1 | 14% |
| | 0.06 | 20 | 1.2 | 1917.5 | 15.7 | | 0.4 | 2.1 | 17% |
| | 0.05 | 20 | 1.0 | 1982.5 | 19.4 | | 0.4 | 2.1 | 20% |
| | 0.05 | 20 | 1.0 | 1892.8 | 18.6 | | 0.3 | 2.1 | 14% |
| | 0.05 | 20 | 1.0 | 1830.4 | 17.9 | | 0.3 | 2.1 | 14% |
| | 0.05 | 20 | 1.0 | 1861.4 | 18.2 | | 0.4 | 2.1 | 17% |
| Example 7 | 0.05 | 20 | 1.0 | 1765.9 | 17.3 | 18.2 | 0.4 | 2.1 | 19% |
| | 0.05 | 20 | 1.0 | 1992.7 | 19.5 | | 0.4 | 2.1 | 19% |
| | 0.05 | 20 | 1.0 | 1880.3 | 18.4 | | 0.4 | 2.1 | 18% |
| | 0.05 | 20 | 1.0 | 1983.8 | 19.4 | | 0.4 | 2.1 | 20% |
| | 0.05 | 20 | 1.0 | 1776.8 | 17.4 | | 0.4 | 2.1 | 20% |
| | 0.05 | 20 | 1.0 | 1755.3 | 17.2 | | 0.4 | 2.1 | 20% |
| Example 8 | 0.05 | 20 | 1.0 | 1723.3 | 16.9 | 17.8 | 0.3 | 2.1 | 14% |
| | 0.05 | 20 | 1.0 | 1773.3 | 17.4 | | 0.5 | 2.1 | 23% |
| | 0.05 | 20 | 1.0 | 1852.4 | 18.2 | | 0.3 | 2.1 | 15% |
| | 0.05 | 20 | 1.0 | 1614.0 | 15.8 | | 0.3 | 2.1 | 14% |
| | 0.05 | 20 | 1.0 | 1937.0 | 19.0 | | 0.4 | 2.1 | 18% |
| | 0.05 | 20 | 1.0 | 1976.1 | 19.4 | | 0.5 | 2.1 | 21% |

(continued)

| | Thickness/mm | Width/mm | Cross-sectional area/$mm^2$ | Maximum force/gf | Tensile strength/MPa | Average tensile strength/MPa | Maximum force distance/mm | Sample height/mm | Percentage elongation |
|---|---|---|---|---|---|---|---|---|---|
| Example 9 | 0.06 | 20 | 1.2 | 1847.5 | 15.1 | 16.9 | 0.4 | 2.1 | 19% |
| | 0.05 | 20 | 1.0 | 1772.3 | 17.4 | | 0.4 | 2.1 | 19% |
| | 0.05 | 20 | 1.0 | 1693.1 | 16.6 | | 0.5 | 2.1 | 24% |
| | 0.05 | 20 | 1.0 | 1931.7 | 18.9 | | 0.3 | 2.1 | 15% |
| | 0.05 | 20 | 1.0 | 1782.5 | 17.5 | | 0.4 | 2.1 | 19% |
| | 0.05 | 20 | 1.0 | 1631.8 | 16.0 | | 0.4 | 2.1 | 18% |
| Example 10 | 0.05 | 20 | 1.0 | 1346.1 | 13.2 | 13.0 | 0.4 | 2.1 | 19% |
| | 0.05 | 20 | 1.0 | 1434.8 | 14.1 | | 0.4 | 2.1 | 19% |
| | 0.06 | 20 | 1.2 | 1365.4 | 11.2 | | 0.3 | 2.1 | 14% |
| | 0.05 | 20 | 1.0 | 1470.5 | 14.4 | | 0.4 | 2.1 | 19% |
| | 0.05 | 20 | 1.0 | 1376.6 | 13.5 | | 0.4 | 2.1 | 19% |
| | 0.05 | 20 | 1.0 | 1203.1 | 11.8 | | 0.3 | 2.1 | 14% |
| Comparative Example 2 | 0.05 | 20 | 1 | 659.3 | 6.5 | 6.5 | 0.2 | 2.1 | 10% |
| | 0.05 | 20 | 1 | 663.1 | 6.5 | | 0.1 | 2.1 | 5% |
| | 0.06 | 20 | 1.2 | 621.0 | 5.1 | | 0.2 | 2.1 | 10% |
| | 0.05 | 20 | 1 | 701.4 | 6.9 | | 0.2 | 2.1 | 10% |
| | 0.05 | 20 | 1 | 722.3 | 7.1 | | 0.2 | 2.1 | 10% |
| | 0.05 | 20 | 1 | 698.0 | 6.8 | | 0.2 | 2.1 | 10% |

The data showed that the brexpiprazole oral soluble films prepared in Examples 1-10 of the present disclosure exhibited good tensile strength, and the film of Comparative Example 2 exhibited relatively poor tensile strength and was relatively brittle.

4. Disintegration time test for brexpiprazole oral thin film formulation

The disintegration time of the brexpiprazole oral thin film formulations prepared in the examples and Comparative Example 2 of the present disclosure was determined. The specific determination method is as follows:
Six drug films were randomly selected from each example. One film was gently placed at a time on a watch glass with a diameter of about 8 cm containing 20 mL of artificial saliva at 37 ± 1 °C. The time until complete disintegration under static conditions was recorded. The results are shown in Table 5.

Table 5. Average disintegration time test for oral thin film formulations of Examples 1-10 and Comparative Example 2

| Example | Average disintegration time (s) |
|---|---|
| Example 1 | 33 |
| Example 2 | 37 |
| Example 3 | 51 |
| Example 4 | 38 |
| Example 5 | 54 |
| Example 6 | 50 |
| Example 7 | 52 |
| Example 8 | 55 |
| Example 9 | 57 |
| Example 10 | 35 |
| Comparative Example 2 | 26 |

Based on the results described above, the oral thin films of the examples and comparative examples of the present disclosure could rapidly disintegrate in a short time.

5. Stability test for brexpiprazole oral thin film formulation

Brexpiprazole oral thin films were prepared according to Example 7 and cut into unit doses of 1 mg and 2 mg. The films were packaged in pharmaceutical composite bags made of polyester/aluminum/polyethylene, and their stability was evaluated under long-term test conditions (25 ± 2 °C/40% RH ± 5% RH) and accelerated test conditions (40 ± 2 °C/75% RH ± 5% RH). The results are as follows:

(1) Detection results for related substances

| Condition | Specification | IM-E (%) | IM-30 (%) | 1M-56 (%) | Maximum unknown individual impurity (%) | Total impurity (%) |
|---|---|---|---|---|---|---|
| 0 day | 1 mg | Undetectable | Undetectable | Undetectable | 0.04 | 0.1 |
| | 2 mg | Undetectable | Undetectable | Undetectable | 0.08 | 0.2 |

(continued)

| Condition | Specification | IM-E (%) | IM-30 (%) | IM-56 (%) | Maximum unknown individual impurity (%) | Total impurity (%) |
|---|---|---|---|---|---|---|
| Accelerated for 6 months | 1 mg | 0.12 | Undetectable | 0.01 | 0.07 | 0.4 |
| | 2 mg | 0.12 | Undetectable | 0.03 | 0.07 | 0.5 |
| Long-term for 6 months | 1 mg | 0.03 | Undetectable | Undetectable | 0.05 | 0.2 |
| | 2 mg | 0.02 | Undetectable | Undetectable | 0.07 | 0.3 |

(2) Dissolution detection results

| Specification | 0 day | Accelerated for 6 months | Long-term for 6 months |
|---|---|---|---|
| 1 mg | 97% | 92% | 94% |
| 2 mg | 90% | 90% | 88% |

(3) Content detection results

| Specification | 0 day | Accelerated for 6 months | Long-term for 6 months |
|---|---|---|---|
| 1 mg | 99.7% | 99.7% | 99.4% |
| 2 mg | 99.2% | 99.4% | 99.1% |

(4) Results of mechanical strength test

| Specification | Item | | 0 day | Accelerated for 6 months | Long-term for 6 months |
|---|---|---|---|---|---|
| 1 mg | Thickness mm | | 0.062 | 0.064 | 0.069 |
| | Group 1 | Width cm | 1.50 | 1.60 | 1.50 |
| | | Cross-sectional area $mm^2$ | 0.925 | 1.029 | 1.040 |
| | | Maximum force gf | 1099.9 | 1315.8 | 1365.8 |
| | | Maximum force distance mm | 1.066 | 0.903 | 0.827 |
| | | Sample height mm | 2.148 | 2.122 | 2.170 |
| | | Tensile strength Mpa | 11.67 | 12.59 | 12.92 |
| | | Percentage elongation % | 49.9 | 42.6 | 38.1 |

(continued)

| Specification | Item | | 0 day | Accelerated for 6 months | Long-term for 6 months |
|---|---|---|---|---|---|
| | Group 2 | Width cm | 1.70 | 1.60 | 1.60 |
| | | Cross-sectional area $mm^2$ | 1.057 | 1.045 | 1.045 |
| | | Maximum force gf | 1248.3 | 1311.0 | 1389.4 |
| | | Maximum force distance mm | 1.102 | 0.940 | 0.964 |
| | | Sample height mm | 2.197 | 2.170 | 2.128 |
| | | Tensile strength Mpa | 11.61 | 12.37 | 13.06 |
| | | Percentage elongation % | 50.2 | 43.3 | 45.5 |
| 2mg | Thickness mm | | 0.063 | 0.071 | 0.076 |
| | Group 1 | Width cm | 2.00 | 2.00 | 2.00 |
| | | Cross-sectional area $mm^2$ | 1.275 | 1.417 | 1.513 |
| | | Maximum force gf | 1386.0 | 1722.9 | 1736.7 |
| | | Maximum force distance mm | 1.308 | 1.000 | 1.132 |
| | | Sample height mm | 2.195 | 2.170 | 2.130 |
| | | Tensile strength Mpa | 10.89 | 11.94 | 11.30 |
| | | Percentage elongation % | 59.8 | 46.1 | 53.3 |
| | Group 2 | Width cm | 2.10 | 2.00 | 2.00 |
| | | Cross-sectional area $mm^2$ | 1.408 | 1.313 | 1.460 |
| | | Maximum force gf | 1431.4 | 1725.2 | 1880.2 |
| | | Maximum force distance mm | 1.454 | 1.033 | 1.096 |
| | | Sample height mm | 2.250 | 2.177 | 2.199 |
| | | Tensile strength Mpa | 9.85 | 12.91 | 12.66 |
| | | Percentage elongation % | 64.5 | 47.5 | 49.8 |

(5) Results of folding endurance test

| Specification | 0 day | Accelerated for 6 months | Long-term for 6 months |
|---|---|---|---|
| 1 mg | > 5 times | > 5 times | > 5 times |
| 1 mg | > 5 times | > 5 times | > 5 times |
| 1 mg | > 5 times | > 5 times | > 5 times |
| 2 mg | > 5 times | > 5 times | > 5 times |
| 2 mg | > 5 times | > 5 times | > 5 times |
| 2 mg | > 5 times | > 5 times | > 5 times |

It can be seen from the above experimental data that the brexpiprazole oral soluble film compositions provided by the present disclosure have the advantages of thin thickness, rapid disintegration, stable properties, good mechanical performance, immediate dissolution in the oral cavity without drinking water, and rapid oral absorption.

The present disclosure overcomes the disadvantages of conventional brexpiprazole tablets in the prior art, which have to be disintegrated in the stomach before drug release, resulting in delayed onset of action, limited bioavailability, inconvenient administration, poor patient compliance, and the like, and provides a brexpiprazole oral thin film formulation,

the preparation method therefor, and use thereof. The brexpiprazole oral thin film formulation of the present disclosure has the advantages of thin thickness, good mouthfeel, stable properties, immediate dissolution in the oral cavity without drinking water, and rapid oral absorption. Furthermore, the preparation process is simple, the drug loading is high, and the drug content uniformity is good, addressing issues such as poor compliance in patients with schizophrenia and cheeking and spitting out of the dosage form, which is particularly suitable for patients with dysphagia.

The embodiments of the present disclosure have been described above. However, the embodiments described above are not intended to limit the present disclosure. Any modification, equivalent replacement, improvement, and the like made without departing from the spirit and principle of the present disclosure shall fall within the claimed scope of the present disclosure.

## Claims

1. A brexpiprazole oral thin film formulation, wherein the brexpiprazole oral thin film formulation comprises the following components: an active pharmaceutical ingredient and a film-forming material, the active pharmaceutical ingredient is 7-[4-(4-benzo[B]thiophen-4-yl-1-piperazine)butoxy]-2(1H)-quinolinone represented by formula I and/or a pharmaceutically acceptable salt thereof, and the active pharmaceutical ingredient has a particle size $D_{90} \leq 70$ μm; the film-forming material is one or more of hydroxypropyl methylcellulose, polyvinyl alcohol, and hydroxypropylcellulose; the brexpiprazole oral thin film formulation does not comprise dimethyl silicone oil;

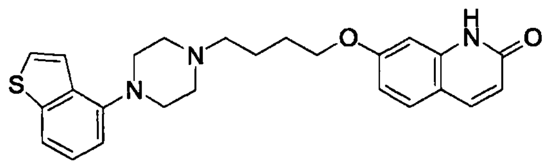

I.

2. The brexpiprazole oral thin film formulation according to claim 1, wherein

   the active pharmaceutical ingredient has a particle size $D_{90} \leq 65.0$ μm or $D_{90} \leq 50.0$ μm;
   and/or,
   the active pharmaceutical ingredient has a mass percentage content of 1.0%-50.0% or 4.0%-40.0%, wherein the mass percentage content refers to a percentage of the mass of the active pharmaceutical ingredient relative to the total mass of the brexpiprazole oral thin film formulation.

3. The brexpiprazole oral thin film formulation according to claim 1, wherein
   the film-forming material has a mass percentage content of 30.0%-80.0% or 40.0%-75.0%, wherein the mass percentage content refers to a percentage of the mass of the film-forming material relative to the total mass of the brexpiprazole oral thin film formulation.

4. The brexpiprazole oral thin film formulation according to claim 1, further comprising one or more of a plasticizer, a sweetener, a disintegrant, a filler, and a colorant.

5. The brexpiprazole oral thin film formulation according to claim 4, wherein

   the plasticizer is selected from one or more of polyethylene glycol, glycerol, propylene glycol, silicone oils other than dimethyl silicone oil, polypropylene glycol, and hexanediol;
   and/or,
   the sweetener is selected from one or more of aspartame, sucralose, fructose, sucrose, stevioside, glycyrrhizin, an essence, a spice, saccharin, and saccharin sodium;
   and/or,
   the disintegrant is selected from one or more of low-substituted hydroxypropylcellulose, crospovidone, croscarmellose sodium, cross-linked sodium carboxymethyl starch, and starch;
   and/or,
   the filler is selected from one or more of microcrystalline cellulose, pregelatinized starch, mannitol, sucrose, glucose, maltose, lactose, sorbitol, xylitol, maltitol, galactitol, erythritol, dextrin, and trehalose;

and/or,
the colorant is selected from one or more of titanium dioxide, a pigment, and a lake.

**6.** The brexpiprazole oral thin film formulation according to claim 4, wherein

the sweetener has a mass percentage content of 0-10.0%, wherein the mass percentage content refers to a percentage of the mass of the sweetener relative to the total mass of the brexpiprazole oral thin film formulation; and/or,
the plasticizer has a mass percentage content of 0-30.0%, wherein the mass percentage content refers to a percentage of the mass of the plasticizer relative to the total mass of the brexpiprazole oral thin film formulation; and/or,
the disintegrant has a mass percentage content of 0-10.0%, wherein the mass percentage content refers to a percentage of the mass of the plasticizer relative to the total mass of the brexpiprazole oral thin film formulation; and/or,
the filler has a mass percentage content of 0-60.0%, wherein the mass percentage content refers to a percentage of the mass of the filler relative to the total mass of the brexpiprazole oral thin film formulation; and/or,
the colorant has a mass percentage content of 0-5.0%, wherein the mass percentage content refers to a percentage of the mass of the colorant relative to the total mass of the brexpiprazole oral thin film formulation;
preferably, the brexpiprazole oral thin film formulation comprises or consists essentially of the following components: brexpiprazole, the film-forming material, the plasticizer, the sweetener, and the colorant, wherein the brexpiprazole has a $D_{90} \leq 65.0$ μm;
preferably, the brexpiprazole oral thin film formulation comprises or consists essentially of the following components:
4.0%-15.0% brexpiprazole, 50.0%-75.0% hydroxypropyl methylcellulose, 10.0%-30.0% glycerol, 5%-10% sweetener, and 0-0.5% colorant, wherein the brexpiprazole has a $D_{90} \leq 65.0$ μm.

**7.** The brexpiprazole oral thin film formulation according to claim 1, wherein the brexpiprazole oral thin film formulation described in the present disclosure is any one of the following formulas:

formula I: 16.7% brexpiprazole, 45.0% hydroxypropyl methylcellulose, 16.7% polyvinyl alcohol, 20.8% glycerol, and 0.8% sucralose, wherein the brexpiprazole has a $D_{90}$ of 27.8 μm;
formula II: 13.8% brexpiprazole, 57.9% hydroxypropyl methylcellulose, 24.1% glycerol, 0.7% stevioside, and 3.5% crospovidone, wherein the brexpiprazole has a $D_{90}$ of 27.8 μm;
formula III: 23.8% brexpiprazole, 47.6% polyvinyl alcohol, 23.8% glycerol, 2.4% stevioside, and 2.4% essence, wherein the brexpiprazole has a $D_{90}$ of 27.8 μm;
formula IV: 4.3% brexpiprazole, 34.8% polyvinyl alcohol, 21.7% hydroxypropylcellulose, 6.5% sucralose, 2.2% essence, and 30.5% microcrystalline cellulose, wherein the brexpiprazole has a $D_{90}$ of 27.8 μm;
formula V: 8.7% brexpiprazole, 65.2% hydroxypropyl methylcellulose, 17.4% glycerol, 4.3% stevioside, 4.3% essence, and 0.1% lake, wherein the brexpiprazole has a $D_{90}$ of 9.7 μm;
formula VI: 8.7% brexpiprazole, 65.2% hydroxypropyl methylcellulose, 17.4% glycerol, 4.3% stevioside, 4.3% essence, and 0.1% lake, wherein the brexpiprazole has a $D_{90}$ of 16.5 μm;
formula VII: 8.7% brexpiprazole, 65.2% hydroxypropyl methylcellulose, 17.4% glycerol, 4.3% stevioside, 4.3% essence, and 0.1% lake, wherein the brexpiprazole has a $D_{90}$ of 27.8 μm;
formula VIII: 8.7% brexpiprazole, 65.2% hydroxypropyl methylcellulose, 17.4% glycerol, 4.3% stevioside, 4.3% essence, and 0.1 % lake, wherein the brexpiprazole has a $D_{90}$ of 35.1 μm;
formula IX: 8.7% brexpiprazole, 65.2% hydroxypropyl methylcellulose, 17.4% glycerol, 4.3% stevioside, 4.3% essence, and 0.1% lake, wherein the brexpiprazole has a $D_{90}$ of 45.6 μm; and
formula X: 40.0% brexpiprazole, 50.0% polyvinyl alcohol, 4.0% glycerol, 2.0% titanium dioxide, and 4.0% crospovidone, wherein the brexpiprazole has a $D_{90}$ of 45.6 μm.

**8.** A preparation method for the brexpiprazole oral thin film formulation according to any one of claims 1-7, wherein the preparation method comprises the following steps:

1) dissolving one or more of water-soluble excipients such as the plasticizer, the sweetener, the disintegrant, the filler, and the colorant in purified water, and adding the film-forming material and dissolving the film-forming material;
2) mixing insoluble excipients in a formulation with the solution obtained in step 1) to obtain a suspension;
3) adding the active pharmaceutical ingredient into the solution obtained in step 2), and mixing uniformly;

4) defoaming the suspension obtained in step 3) to obtain a defoamed suspension; and
5) coating a substrate with the defoamed suspension obtained in step 4), drying, and forming a film to obtain the brexpiprazole oral thin film formulation.

**9.** Use of the brexpiprazole oral thin film formulation according to any one of claims 1-8 for manufacturing a medicament for the treatment of a central nervous system disease.

**10.** The use according to claim 9, wherein the central nervous system disease is major depressive disorder or schizophrenia.

**INTERNATIONAL SEARCH REPORT**

International application No.
**PCT/CN2024/119447**

**A. CLASSIFICATION OF SUBJECT MATTER**

A61K9/70(2006.01)i; A61K31/496(2006.01)i; A61K47/38(2006.01)i; A61K47/10(2017.01)i; A61P25/24(2006.01)i; A61P25/18(2006.01)i

According to International Patent Classification (IPC) or to both national classification and IPC

**B. FIELDS SEARCHED**

Minimum documentation searched (classification system followed by classification symbols)

IPC: A61K A61P

Documentation searched other than minimum documentation to the extent that such documents are included in the fields searched

Electronic data base consulted during the international search (name of data base and, where practicable, search terms used)

CNTXT; DWPI; ENTXT; PUBMED; ELSEVIER; NCBI; VCN; Web of Science; STNext; CJFD; CNKI; 万方, WANFANG; 读秀, DUXIU: 薄膜, 膜剂, 布瑞哌唑, 依匹唑派, 依匹哌唑, 布雷帕唑, 依匹唑哌, 依匹派唑, 普瑞哌唑, 粒径, 聚乙烯醇, 羟丙甲纤维素, 羟丙基甲基纤维素, 羟丙甲基纤维素, 羟丙基纤维素, 羟丙纤维素, 二甲基硅油, 二甲硅油, 聚乙二醇, 甘油, 丙二醇, 硅油, 聚丙二醇, 己二醇, 阿司帕坦, 三氯蔗糖, 果糖, 蔗糖, 甜菊糖苷, 甘草甜素, 香精, 香料, 糖精, 糖精钠, 低取代羟丙基纤维素, 交联聚维酮, 交联羧甲基纤维素钠, 交联羧甲基淀粉钠, 淀粉, 微晶纤维素, 预胶化淀粉, 甘露醇, 蔗糖, 葡萄糖, 麦芽糖, 乳糖, 山梨醇, 木糖醇, 麦芽糖醇, 半乳糖醇, 赤藓糖醇, 糊精, 海藻糖, 二氧化钛, 色素, 色淀, REXULTI, Brexpiprazole, Quinolinone, OPC-34712, 913611-97-9 , Hyprolose, HP, HPC, D90, Polyvinyl alcohol, hypromellose, hydroxypropyl methylcellulose, simethicone, dimethicone, polyethylene glycol, PEG, glycerol, propylene glycol, silicone oil, polypropylene glycol, hexanediol, aspartame, sucralose, fructose, sucrose, steviol glycosides, glycyrrhizin, fragrances, Flavors, Saccharin, Sodium Saccharin, Crospovidone, Croscarmellose Sodium, Croscarmellose Sodium, Starch, Microcrystalline Cellulose, Pregelatinized Starch, Mannitol, Sucrose, Glucose, Maltose, Lactose, Sorbitol, Xylitol, Maltitol, Galactitol, Erythritol, Dextrins, Trehalose, Titanium Dioxide, Pigments, Lakes, 结构式检索, structural formula search

**C. DOCUMENTS CONSIDERED TO BE RELEVANT**

| Category* | Citation of document, with indication, where appropriate, of the relevant passages | Relevant to claim No. |
|---|---|---|
| X | CN 114767663 A (ZHEJIANG HEZE PHARMACEUTICAL TECHNOLOGY CO., LTD. et al.) 22 July 2022 (2022-07-22)<br>description, paragraphs [0030]-[0035], and claim 10 | 1-6, 8-10 |
| X | CN 105395528 A (BEIJING KANGLISHENG PHARMACEUTICAL TECHNOLOGY DEVELOPMENT CO., LTD.) 16 March 2016 (2016-03-16)<br>claims 1-6 | 1-6, 8-10 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **04 December 2024** | **09 December 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

## INTERNATIONAL SEARCH REPORT

International application No.
**PCT/CN2024/119447**

### C. DOCUMENTS CONSIDERED TO BE RELEVANT

| Category* | Citation of document, with indication, where appropriate, of the relevant passages | Relevant to claim No. |
|---|---|---|
| A | CN 115252585 A (SHANGHAI BOCIMED PHARMACEUTICAL CO., LTD.) 01 November 2022 (2022-11-01)<br>claims 1-10 | 1-10 |
| A | CN 115192549 A (SHANGHAI BOCIMED PHARMACEUTICAL CO., LTD.) 18 October 2022 (2022-10-18)<br>claims 1-10 | 1-10 |
| A | CN 114767663 A (ZHEJIANG HEZE PHARMACEUTICAL TECHNOLOGY CO., LTD. et al.) 22 July 2022 (2022-07-22)<br>description, paragraphs [0030]-[0035], and claim 10 | 7 |

Form PCT/ISA/210 (second sheet) (July 2022)

**INTERNATIONAL SEARCH REPORT**
**Information on patent family members**

International application No.
**PCT/CN2024/119447**

| Patent document cited in search report | Publication date (day/month/year) | Patent family member(s) | Publication date (day/month/year) |
|---|---|---|---|
| CN 114767663 A | 22 July 2022 | None | |
| CN 105395528 A | 16 March 2016 | None | |
| CN 115252585 A | 01 November 2022 | WO 2022218357 A1 | 20 October 2022 |
| | | TW 202245770 A | 01 December 2022 |
| | | TWI 835118 B | 11 March 2024 |
| CN 115192549 A | 18 October 2022 | TW 202245771 A | 01 December 2022 |
| | | TWI 820674 B | 01 November 2023 |
| | | WO 2022218358 A1 | 20 October 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 202311227319 A
- CN 105078910 A
- CN 105395528 A
- CN 115192549 A